# DEMANDE DE BREVET EUROPEEN

(11) **EP 2 923 728 A1**
(43) Date de publication de la demande: **30.09.2015**
(21) Numéro de dépôt: 15157714.5
(22) Date de dépôt: 05.03.2015
(51) Int. Cl.: A61N 5/06

(54) **Dispositif de traitement par photothérapie**

(30) Priorité: 24.03.2014 FR 1452442
(71) Demandeur: Lucibel SA, 92500 Rueil Malmaison (FR)
(72) Inventeur: Houot, Jean-Laurent, 38110 Saint-Clair-de-la-Tour (FR)
(74) Mandataire: Martin, Marie-Aude

(57) **Abrégé**

L'équipement (10) de traitement comprend une source (30) de génération d'un flux lumineux et des moyens (34) de pilotage de la source selon un programme de pilotage prédéfini. Plus particulièrement, l'équipement comprend une pluralité de modules portatifs (18, 20), chaque module (18, 20) comprenant un boîtier propre à être saisi par l'utilisateur. La pluralité de modules comprend en outre un module de commande (20) et au moins un module d'émission de lumière (18) comprenant la source (30) et les moyens de pilotage (34). Par ailleurs, le module de commande (20) comprend des moyens aptes à générer des signaux de commande de déclenchement de l'exécution du programme de pilotage et de l'extinction de la source de lumière (30) par les moyens de pilotage (34).

## Description

La présente invention concerne un dispositif de traitement d'une région externe du corps d'un être vivant par photothérapie. Elle s'applique plus particulièrement mais non exclusivement au traitement de la peau du corps ou du visage d'un patient humain.

La photothérapie consiste en l'application d'un rayonnement lumineux présentant un spectre d'émission prédéfini directement sur la région du corps à traiter.

Le rayonnement est de préférence sensiblement monochromatique ou présente un spectre d'émission compris dans une bande étroite de longueurs d'onde. Généralement, le spectre d'émission comprend une longueur d'onde maximale située dans la lumière visible rouge ou encore dans la lumière visible bleue, ces longueurs d'onde ayant démontré une efficacité particulière pour le traitement de certaines affections de la peau.

Il a été ainsi observé au cours de nombreuses études scientifiques, qu'un traitement par photothérapie dans une plage de longueurs d'onde prédéfinie permet d'agir de façon bénéfique sur de nombreux problèmes cutanés, tels que les cicatrices, les vergetures, l'acné, les rides et ridules, mais également certains cancers cutanés.

Plus particulièrement, lors de l'application du rayonnement sur la peau, notamment dans la lumière rouge et plus particulièrement autour de la longueur d'onde 630 nm, les cellules de la région irradiées vont réagir selon un principe similaire à celui de la photosynthèse des végétaux.

Cette lumière va stimuler les cellules, et plus spécifiquement des structures cellulaires essentielles à leur fonctionnement telles que les mitochondries.

De façon connue en soi, les mitochondries jouent un rôle particulièrement important dans le processus de respiration cellulaire ainsi que dans le processus de transformation des nutriments apportés à la cellule en un vecteur énergétique désigné couramment par ATP (acronyme pour Adénosine Tri Phosphate). Dès lors, la stimulation des mitochondries par cette lumière rouge contribue à une régénération des cellules par une amélioration du processus de respiration et d'alimentation des cellules.

Cette régénération cellulaire va se manifester par exemple pour les cellules du visage par une augmentation de la production de collagène et d'élastine et par conséquent une diminution des rides et ridules.

Ce traitement par photothérapie peut être appliqué également en combinaison avec un produit photo-sensibilisant appliqué sur la région à traiter quelques heures avant son exposition au rayonnement lumineux pour augmenter l'efficacité du traitement. On parle alors dans ce cas de photothérapie dynamique (connue également sous l'acronyme anglais PDT « Photo Dynamic Therapy »).

Il est déjà connu dans l'état de la technique un équipement de photothérapie à usage professionnel comprenant un support monté sur un socle à roulettes et une tête articulée sur le support comprenant quatre panneaux lumineux adjacents. Chaque panneau lumineux comprend une surface d'émission formée par une matrice d'un peu moins de mille LEDS (acronyme anglais pour Light Emitting Device). En utilisation, le patient s'installe sur une chaise ou éventuellement une couchette et la tête de l'équipement peut être orientée dans la direction de la région à traiter.

L'inconvénient d'un tel équipement est qu'il est encombrant et qu'il a un poids important qui ne permet pas un déplacement aisé de l'équipement (environ une vingtaine de kilogrammes). Cette mise en oeuvre complexe est particulièrement désavantageuse lorsque la surface à traiter a des dimensions petites.

L'invention vise à résoudre ce problème en proposant de simplifier la procédure de mise en place du traitement.

A cet effet, l'invention a pour objet un équipement de traitement d'une région du corps d'un utilisateur par photothérapie, du type comprenant une source de génération d'un flux lumineux et des moyens de pilotage de la source selon un programme de pilotage prédéfini, caractérisé en ce qu'il comprend une pluralité de modules portatifs, chaque module comprenant un boîtier propre à être saisi par l'utilisateur, comprenant un module de commande et au moins un module d'émission de lumière comprenant la source et les moyens de pilotage **et en ce que** le module de commande comprend des moyens aptes à générer des signaux de commande de déclenchement de l'exécution du programme de pilotage et de l'extinction de la source lumineuse par les moyens de pilotage.

En réalisant un équipement formé d'une pluralité de modules, la surface du corps du patient à traiter peut être échantillonnée. Les modules couvrent alors la surface à traiter en épousant la forme du corps. Par exemple, les modules peuvent couvrir aussi bien une zone ventrale ou encore frontale, dorsale ou autres en suivant les courbes du corps. Par ailleurs, lorsque la surface a une dimension réduire, l'aspect modulaire permet d'adapter le nombre de modules. Ceci permet de simplifier la mise en oeuvre de l'équipement en l'adaptant aux besoins.

Par ailleurs, l'invention propose d'utiliser une communication de type maître-esclave. En centralisant les moyens de commande dans un seul module « maître », il est possible de piloter l'ensemble des autres modules « esclaves » simultanément. La mise en oeuvre de l'équipement s'en trouve alors simplifiée.

Selon un mode de réalisation particulier de l'invention, les moyens de pilotage comprennent des moyens de régulation d'une intensité d'un courant traversant la source de lumière.

De façon plus générale, le dispositif qui permet l'alimentation d'une diode électroluminescente à partir d'une tension d'alimentation générée par une source est appelé communément un « driver ». Le « driver » permet de générer un courant continu dans le sens passant de la diode.

De préférence, la source de génération d'un flux lumineux comprend une pluralité d'unités d'éclairage, chaque unité comprenant au moins une puce diode électroluminescente, les unités étant montées en série.

De préférence, lorsque deux modules de lumière sont raccordés entre eux, les sources de génération d'un flux lumineux sont montées en parallèle.

Dans un mode de réalisation préféré, les modules sont liés électriquement entre eux pour permettre le passage d'un courant électrique entre les modules.

De préférence, les modules comprennent une liaison mécanique libérable de type à aimantation afin de former une liaison magnétique amovible entre les modules.

Dans un mode de réalisation préféré, le module de commande comprend une source d'alimentation électrique reliée à un commutateur marche/arrêt d'activation/désactivation de la source d'alimentation électrique, les signaux de commande étant générés par l'activation ou la désactivation de la source d'alimentation électrique.

De préférence, le module de commande comprend des moyens d'ouverture d'un circuit d'alimentation électrique raccordée à la source d'alimentation électrique lorsque les modules lumineux n'émettent pas de lumière.

Dans un mode de réalisation préféré, les modules sont aptes à échanger des données par des moyens de communication de données et dans lequel le module de commande comprend des moyens de mémorisation d'un autre programme de traitement prédéfini et des moyens de communication de l'autre programme aux moyens de pilotage des autres modules.

De préférence, le module de commande comprend des moyens d'acquisition d'un autre programme de pilotage accessible via un appareil externe, tel qu'un terminal mobile.

De préférence, la source de lumière comprend un spectre d'émission ayant une longueur d'onde maximale d'émission choisies parmi les plages de longueurs d'onde 400 nm à 440 nm, 590 nm à 650 nm et 810 nm à 850 nm.

Dans un mode de réalisation préféré, le module de commande comprend une source d'alimentation électrique portative pouvant stocker de l'énergie, en particulier la source est choisie parmi une batterie et une pile à combustible.

De préférence, le module de commande comprend des moyens de communication de données sans fil ou avec fil pour échanger des données avec un appareil externe.

Dans un mode de réalisation préféré, les moyens de pilotage comprennent un microcontrôleur.

De préférence, le programme de pilotage comprend des instructions de code pour la réalisation d'étapes choisies parmi une étape d'allumage de la source de lumière, une étape d'extinction de la source de lumière, une étape de définition d'un mode d'allumage pulsé ou continu de la source de lumière, une étape de définition d'une durée de période d'allumage et une étape de réglage de la puissance d'émission de la source de lumière.

Dans un mode de réalisation préféré, les moyens de pilotage sont aptes à éteindre la source de lumière en cas d'absence de contact d'une partie du module d'émission de lumière sur un objet externe.

D'autres caractéristiques et avantages de l'invention apparaîtront à la lumière de la description qui suit, faite en référence aux dessins annexés dans lesquels :
- la figure 1 représente un équipement de traitement par photothérapie dans une position d'utilisation ;
- la figure 2 représente une vue en perspective de l'équipement de la figure 1 ;
- la figure 3 représente un premier mode de réalisation de l'équipement représenté par la figure 1 ;
- la figure 4 représente un second mode de réalisation de l'équipement représenté par la figure 2 ;
- la figure 5 représente une étape d'un procédé de mise en oeuvre de l'équipement de la figure 4,
- la figure 6 représente un schéma électrique des moyens de pilotage d'un équipement de la figure 1 ;
- la figure 7 représente un schéma électrique des moyens à détrompage des moyens de pilotage de la figure 6.

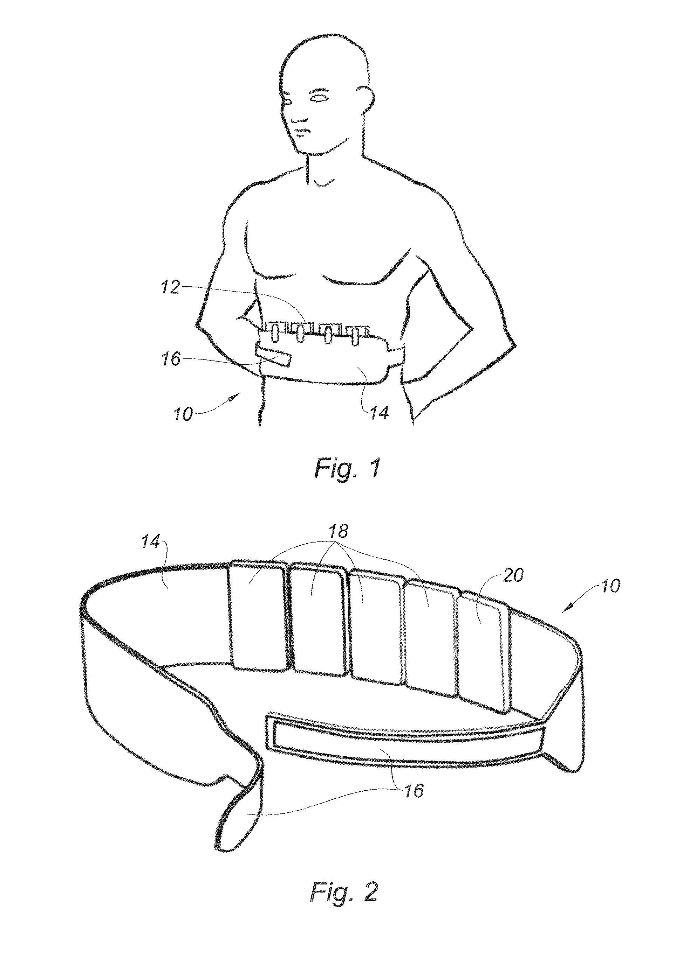

On a représenté sur **la** **figure 1** un équipement de photothérapie selon l'invention. Cet équipement est désigné par la référence générale 10.

Cet équipement comprend comme cela est illustré sur **les** **figures 1 à 4****,** une pluralité de modules 12 portatifs. De préférence, les modules 12 comprennent chacun un boîtier propre à être saisi par un utilisateur.

Dans l'exemple illustré sur **les** **figures 1 et 2****,** les modules 12 sont portés par une ceinture 14 dimensionnée pour enserrer une partie du corps d'un utilisateur, ici, la partie ventrale. La ceinture 14 comprend par exemple des moyens 16 auto-grippant de type velcro ® permettant un ajustement de la ceinture à la dimension de la partie à enserrer. Bien entendu, dans une variante non illustrée, d'autres moyens de fixation à la ceinture peuvent convenir.

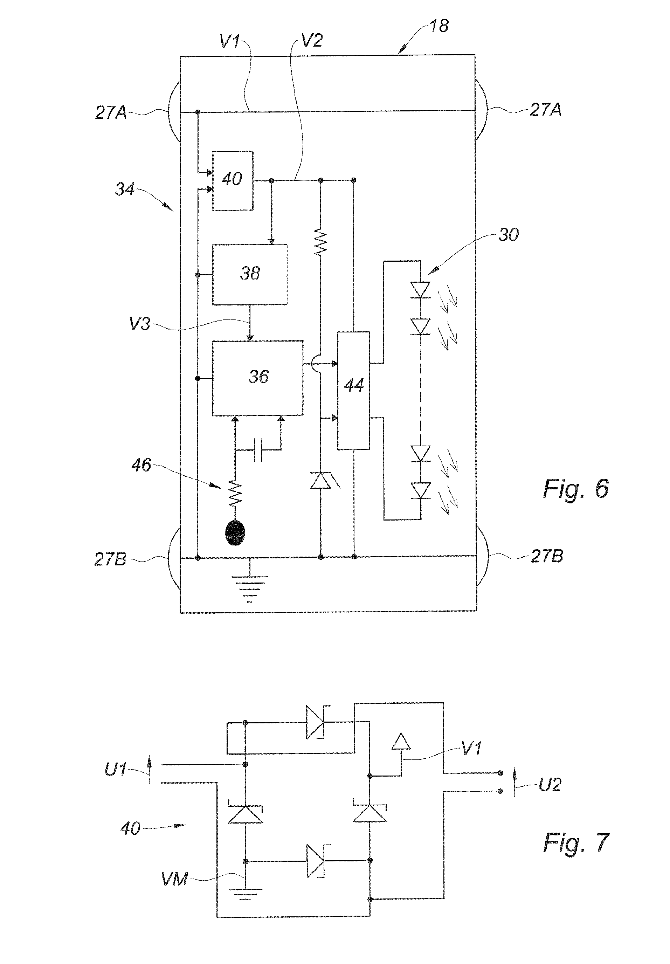

De préférence, les modules 12 sont liés électriquement entre eux pour permettre le passage d'un courant électrique entre les modules. Par ailleurs, à cet effet, les modules 12 comprennent par exemple une liaison mécanique libérable de type à aimantation afin de former une liaison magnétique amovible entre les modules 12. Dans un mode de réalisation préféré, les liaisons magnétiques sont également électriquement conductrices. Par conséquent, la mise en oeuvre de l'équipement 10 se limitera à assembler les modules 12 entre eux par aimantation sans nécessiter de branchements électriques supplémentaires. Dans cet exemple, la liaison magnétique est formée par des moyens de liaison complémentaires 27A, 27B illustrés de façon schématique sur la figure 6. Ces moyens de liaison sont de préférence de type à liaison pivot.

L'équipement 10 comprend parmi les modules 12 au moins un module 18 d'émission de lumière dit module lumineux et au moins un module 20 de commande des modules lumineux 18. Dans l'exemple illustré par **la** **figure 2****,** l'équipement 10 comprend quatre modules lumineux 18 et un module de commande 20.

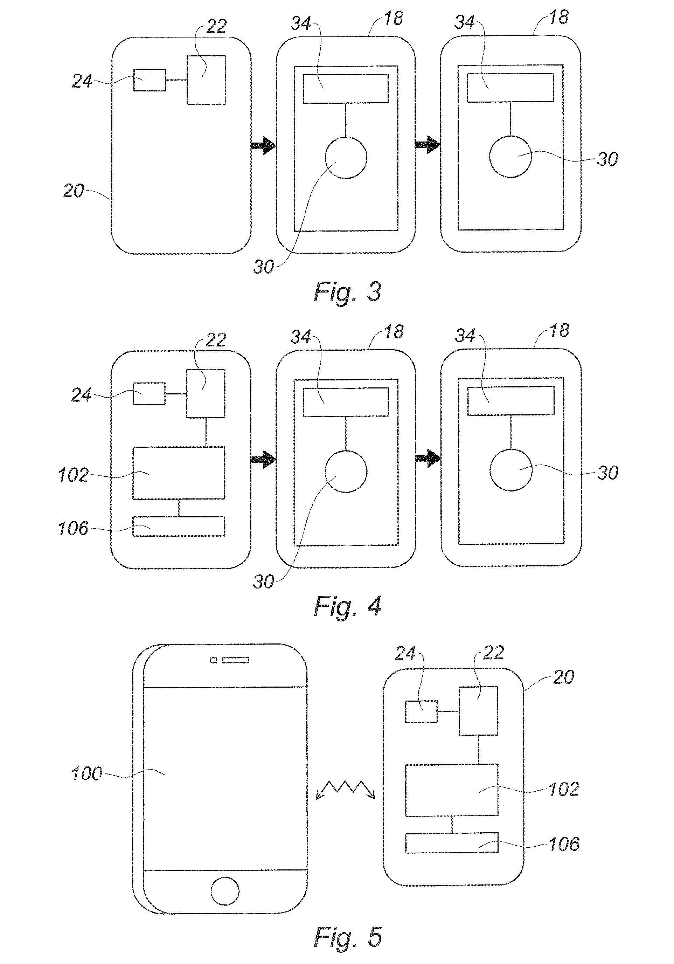

De préférence, en référence **aux** **figures 3 et 4****,** le module de commande 20 comprend une source 22 d'alimentation électrique reliée à un commutateur externe 24 marche/arrêt d'activation/désactivation de la source d'alimentation électrique 22. Ce commutateur 24 peut comprendre un bouton poussoir ou un bouton tactile ménagé sur un côté du boîtier du module de commande 20.

Lorsque le commutateur 24 est dans la position marche, la source d'alimentation 22 permet l'alimentation d'un organe externe, tel qu'un ou plusieurs modules lumineux 18. Dans le cas contraire, lorsque le commutateur est dans la position arrêt, le ou les modules lumineux 18 ne sont plus alimentés.

Dans un mode de réalisation préféré, la source d'alimentation électrique 22 est rechargeable. Par exemple, le module de commande 20 comprend une source d'alimentation électrique 22 portative pouvant stocker de l'énergie, en particulier la source 22 est choisie parmi une batterie et une pile à combustible.

La source d'alimentation électrique 22 est raccordée électriquement à deux bornes d'alimentation électrique du ou des modules lumineux 18. Ces bornes sont respectivement à un potentiel V1 d'alimentation et à un potentiel de référence VM. Le potentiel V1 a une valeur par exemple de 18V. Ces bornes sont aptes à venir en contact avec des bornes complémentaires du module lumineux 18 pour former la liaison mécanique libérable électriquement conductrice. Par exemple, les bornes sont aimantées.

Par ailleurs, chaque module lumineux 18 comprend une source de lumière 30 telle que cela est illustré de façon très schématique sur **les** **figures 3 et 4****.** Le module lumineux 18 comprend une fenêtre d'émission et la source de lumière 30 est apte à éclairer un objet externe, à travers la fenêtre d'émission.

La source de lumière 30 comprend dans cet exemple une pluralité d'unités d'éclairage 32, chaque unité d'éclairage comprenant au moins une puce à diode électroluminescente. De préférence, la source 30 formée de la pluralité d'unités lumineuses est portée par un support de circuit imprimé. Par exemple, les unités lumineuses sont raccordées électriquement entre elles par des interconnexions déposées sur ce support.

Avantageusement, les interconnexions réalisent un branchement en série des unités lumineuses. Ce branchement permet avantageusement d'avoir seulement deux bornes d'entrée et sortie pour l'alimentation électrique de la source de lumière 30. De préférence, la source de lumière 30 comprend un spectre d'émission ayant une longueur d'onde maximale d'émission choisies parmi les plages de longueurs d'onde 400 nm à 440 nm, 590 nm à 650 nm et 810 nm à 850 nm.

Conformément à l'invention, l'équipement 10 comprend également des moyens 34 de pilotage de la source de lumière 30 en fonction d'un programme prédéfini de pilotage de la source 30. La source 30 et ses moyens de pilotage 34 sont regroupés de préférence dans un même module lumineux 18.

De préférence, le programme de pilotage comprend des instructions pour la réalisation d'étapes choisies parmi une étape d'allumage de la source 30, une étape d'extinction de la source 30, une étape de définition d'un mode d'allumage pulsé ou continu de la source 30, une étape de définition d'une durée de période d'allumage, une étape de changement d'un spectre d'émission de la source 30 et une étape de réglage de la puissance d'émission de la source lumineuse 30. Ce pilotage est réalisé de préférence par un pilotage en courant de la source lumineuse 30.

On va maintenant décrire plus en détail les moyens de pilotage de la source de lumière 30 en référence à **la** **figure 6****.**

De préférence, les moyens de pilotage 34 comprennent un processeur. On entendra au sens de l'invention par processeur n'importe quel système apte à traiter et/ou générer des signaux électriques. Dans cet exemple, le processeur comprend un microcontrôleur 36. Ce microcontrôleur 36 comprend notamment une mémoire apte à mémoriser le programme de pilotage. Toutefois, en variante, le processeur peut comporter un microprocesseur, un ASIC (de l'acronyme anglais, « application specific integrated circuit »). Selon **la** **figure 6****,** le microcontrôleur 36 est alimenté en tension par une tension d'alimentation U3 correspondant à la différence de potentiel entre un potentiel V3 et le potentiel de référence noté VM. Cette tension U3 a par exemple une valeur de 3V.

A cet effet, pour passer de la tension U1 fournie par la batterie à la tension U3 d'alimentation du microcontrôleur 36, les moyens de pilotage 34 comprennent également des moyens 38 de conversion d'une tension de valeur plus élevée en une tension de valeur plus faible. Les moyens de conversion 38 comprennent dans cet exemple un « convertisseur buck ». Un convertisseur Buck, désigné également par hacheur série, est une alimentation à découpage qui convertit une tension continue en une autre tension continue de plus faible valeur.

Par ailleurs, les moyens de pilotage 34 comprennent des moyens 40 à détrompage électronique. Ces moyens à détrompage 40 sont aptes à éviter tout court-circuit dans le cas où les modules lumineux 18 seraient assemblés dans un sens contraire. Ces moyens à détrompage 40 comprennent par exemple un circuit à diode 42 configuré pour fournir une polarité identique en sortie quelle que soit la polarité fournie en entrée. Un tel circuit à diode 42 est illustré par exemple sur **la** **figure 7****.**

Dans cet exemple, les moyens de détrompage 40 reçoivent en entrée la tension U1 ou (-U1) si le module lumineux 18 est assemblé dans le mauvais sens) générée par le module d'alimentation électrique 20 et fournissent en sortie la tension U2 de polarité constante quelle que soit la polarité de la tension reçue en entrée. La tension U2 a une valeur absolue égale à la valeur de la tension U1 et a dans cet exemple la valeur 18V. La tension U2 est fournie en entrée des moyens convertisseurs 38 qui génèrent en sortie la tension U3.

Par ailleurs, les moyens de pilotage 34 comprennent des moyens 44 de régulation de l'intensité du courant traversant la source de lumière. Ces moyens de régulation permettent notamment de fournir un courant constant pour alimenter la source lumineuse 30.

Ces moyens de régulation 44 comprennent par exemple un « driver » permettant de fournir un courant d'intensité sensiblement constante à la source de lumière comprenant des diodes électroluminescentes.

Par ailleurs, de préférence, le module lumineux 18 comprend un organe de sécurité 46 apte à provoquer l'extinction de la source de lumière 30 en cas d'absence de contact d'une partie du module lumineux 18 avec un objet externe, notamment un utilisateur. Par exemple, la partie du module 18 correspond à une partie de la fenêtre d'émission de lumière du module 18. En outre, l'organe de sécurité 46 est apte à provoquer l'allumage de la source 30 lorsqu'au moins une première condition liée à la présence d'un contact avec la partie du module.

De préférence, afin de réaliser cette fonction de sécurité, l'organe de sécurité comprend un capteur tactile et une surface tactile liée au capteur. La surface tactile s'étend au moins partiellement sur la fenêtre d'émission.

Un capteur tactile, dit également capacitif, permet notamment de détecter si une personne touche ou effleure un objet. Un tel capteur comporte une ou plusieurs électrodes reliées à un circuit de détection de la capacitance de la ou des électrodes. Lorsqu'une personne touche ou effleure l'électrode, la capacitance de l'électrode charge et se trouve détectée par le circuit de détection qui est apte à délivrer un signal de sortie représentant l'information.

Par exemple, le capteur comprend au moins une électrode reliée au circuit de détection. Cette électrode peut comporter une forme générale de rectangle, trapèze, cercle, ellipse, triangle ou toute autre formes géométriques ou non. Par ailleurs, l'électrode peut aussi avoir une pluralité d'ouvertures ou peut présenter un motif quadrillé.

Par exemple, le circuit de détection peut comporter une puce électronique de circuit intégré. Bien entendu, le circuit de détection peut être réalisé au moyen de composants électroniques discrets. Ce circuit de détection est par exemple apte à détecter une modification d'un champ électrique lié à l'électrode.

Par ailleurs, le capteur peut également comporter une unique électrode ou même plusieurs autres électrodes sans que cela ne sorte du cadre d'invention.

On notera que, de préférence, lorsque deux modules d'émission de lumière 18 sont raccordés entre eux, les sources de génération d'un flux lumineux de chacun des modules 18 sont montées en parallèle. Ceci permet de limiter l'intensité du courant circulant entre les modules.

De préférence, le module de commande 20 comprend des moyens d'ouverture d'un circuit d'alimentation électrique raccordée à la source d'alimentation électrique lorsque les modules lumineux n'émettent pas de lumière. Conformément à l'invention, le module de commande 20 comprend des moyens aptes à générer des signaux de commande de déclenchement de l'exécution du programme de pilotage et de l'extinction de la source lumineuse par les moyens de pilotage. Le déclenchement de l'exécution du programme de pilotage et l'extinction des sources lumineuses 30 sont opérés par des moyens de commandes communs à l'ensemble des modules lumineux 18.

Dans le premier mode de réalisation illustré par **la** **figure 3****,** les signaux de commande sont générés par l'activation ou la désactivation de la source d'alimentation électrique 22.

Dans ce premier mode de réalisation, le microcontrôleur 36 comprend le programme de traitement mémorisé et dès lors qu'il est alimenté, dans cet exemple par la tension U3, il exécute le programme.

Lorsque le module lumineux 18 est alimenté électriquement, les moyens de pilotage 34 s'activent, en particulier le microcontrôleur 36 est alimenté et exécute le programme de pilotage de la source de lumière 30. Lorsque le module lumineux 18 n'est plus alimenté électriquement, les moyens de pilotage 34 et la source de lumière 30 ne sont plus alimentés et la source 30 est éteinte.

Dans un second mode de réalisation de l'invention illustré par **les** **figures 4 et 5****,** les modules 18 et 20 sont aptes à échanger des données par des moyens de communication de données (non représentés).

Le module de commande 20 comprend en outre dans ce cas des moyens de mémorisation 106 d'un autre programme de traitement prédéfini et des moyens de communication de l'autre programme aux moyens de pilotage 34 des autres modules lumineux 18. Dans ce cas, le microcontrôleur 36 de chaque module de lumière 18 est apte à mémoriser le nouveau programme transmis par le module de commande 20.

Par exemple, les modules 18, 20 échangent des données par courant porteur, avantageusement par la liaison magnétique électriquement conductrice. En variante, les modules 18, 20 échangent des données par communication sans fil tel que par communication de type Bluetooth, par communication de type en champ proche, de type WIFI, etc.

De préférence, le module de commande 20 comprend des moyens 102 d'acquisition d'un autre programme de pilotage accessible via un appareil externe, tel qu'un terminal mobile 100. Dans cet exemple, les moyens 102 d'acquisition comprennent un processeur, par exemple un microcontrôleur. Les moyens d'acquisition 102 et les moyens de mémorisation 106 peuvent être formés par un même microcontrôleur.

Le module de commande 20 comprend par exemple des moyens de communication de données sans fil ou avec fil pour échanger des données avec l'appareil externe 100. Le module de commande 20 récupère un nouveau programme de pilotage et peut le transmettre aux modules lumineux grâce aux moyens d'acquisition 102 et aux moyens de communication avec le terminal 100.

Sur **la** **figure 5****,** on a représenté le module de commande 20 avec un terminal mobile 100 en cours d'échange de données grâce aux moyens de communication.

Par ailleurs, dans ce second mode de réalisation, le module de commande 20 peut comprendre une interface d'utilisateur. Cette interface utilisateur peut être utilisée par exemple pour sélectionner un programme de pilotage mémorisé dans les moyens de mémorisation 106.

On va maintenant décrire en référence aux figures les principaux aspects de fonctionnement de l'équipement selon l'invention.

Dans un premier temps, l'utilisateur assemble les modules 18, 20 entre eux par aimantation. Il peut choisir autant de modules lumineux 18 qu'il est nécessaire pour couvrir la dimension de la surface à traiter, dans cet exemple, la zone ventrale. Toutefois, dans cet exemple, il est souhaitable de limiter le nombre de modules lumineux 18 à cinq par module d'alimentation électrique 20.

Il dispose ces différents modules 18, 20 sur la ceinture. Puis il appuie sur le bouton marche du module de commande 20.

Si les modules lumineux 18 sont bien positionnés, l'organe de sécurité 46 va autoriser l'allumage des modules lumineux 18. Si un des modules lumineux 18 n'est pas positionné correctement contre la surface de peau de l'utilisateur, l'organe de sécurité 46 du module 18 va provoquer l'extinction de la source lumineuse 30 de ce dernier. Ceci permet avantageusement d'éviter toute exposition accidentelle au rayonnement émis par un module lumineux 18 mal positionné.

Selon le premier mode de réalisation, l'alimentation du microcontrôleur 36 de chaque module 18 permet l'exécution du programme de pilotage mémorisé. Le programme comprend notamment une durée de pilotage au cours de laquelle le rayonnement émis peut suivre une session particulière comprenant des sous-périodes d'allumage pulsée ou continue de la source de lumière 30.

Dans le cas où l'utilisateur souhaite raccourcir la durée du traitement, il lui suffit de placer le commutateur du module de commande 20 dans la position arrêt ce qui permet de provoquer l'extinction de tous les modules de lumière 18.

Dans le second mode de réalisation, avantageusement, l'utilisateur peut choisir une session de traitement parmi différentes sessions de traitement disponibles sur un appareil externe. Dans ce mode de réalisation, l'utilisateur récupère un nouveau programme par l'intermédiaire de son terminal mobile 100. Ce dernier comprend une application pour terminal mobile (de type « apps ») permettent de sélectionner un programme de traitement prédéfini permettant le traitement d'une pathologie ou affection particulière.

Le module de commande 20, grâce à ses moyens d'acquisition 102, récupère le nouveau programme et le mémorise. Puis lors de l'assemblage des modules 18 à 20 entre eux, le nouveau programme est transféré aux moyens de pilotage des modules lumineux 18.

Cette étape de transfert peut être réalisée initialement lors de la mise en marche du module de commande 20. Ce nouveau programme est alors mémorisé dans chaque microcontrôleur 36 puis est exécuté. Les étapes successives de transfert et d'exécution du programme peuvent être avantageusement déclenchées grâce à l'interface utilisateur disposée par exemple sur le module de commande.

Par ailleurs, en variante, le module de commande 20 peut comprendre dans sa mémoire une pluralité de programmes de pilotage différents préenregistrés et l'interface utilisateur peut permettre une sélection d'un programme parmi la pluralité de programmes préenregistrés.

Bien entendu, d'autres modes de réalisation sont envisageables sans sortir du cadre de l'invention. Ainsi, diverses modifications peuvent être apportées par l'homme du métier à l'invention qui vient d'être décrite à titre d'exemple.

## Revendications

1. Equipement (10) de traitement d'une région du corps d'un utilisateur par photothérapie, du type comprenant une source (30) de génération d'un flux lumineux et des moyens (34) de pilotage de la source selon un programme de pilotage prédéfini, **caractérisé en ce qu'il** comprend une pluralité de modules portatifs (18, 20), chaque module (18, 20) comprenant un boîtier propre à être saisi par l'utilisateur, comprenant un module de commande (20) et au moins un module d'émission de lumière (18) comprenant la source (30) et les moyens de pilotage (34) **et en ce que** le module de commande (20) comprend des moyens aptes à générer des signaux de commande de déclenchement de l'exécution du programme de pilotage et de l'extinction de la source de lumière (30) par les moyens de pilotage (34).

2. Equipement (10) selon la revendication précédente, dans lequel les moyens de pilotage (34) comprennent des moyens (44) de régulation d'une intensité d'un courant traversant la source de lumière (30).

3. Equipement (10) selon l'une quelconque des revendications précédentes, dans lequel la source (30) de génération d'un flux lumineux comprend une pluralité d'unités d'éclairage, chaque unité comprenant au moins une puce diode électroluminescente, les unités étant montées en série.

4. Equipement (10) selon l'une quelconque des revendications précédentes, dans lequel lorsque deux modules de lumière (18) sont raccordés entre eux, les sources de génération d'un flux lumineux (30) sont montées en parallèle.

5. Equipement (10) selon l'une quelconque des revendications précédentes, dans lequel les modules (18, 20) sont liés électriquement entre eux pour permettre le passage d'un courant électrique entre les modules (18, 20).

6. Equipement (10) selon l'une quelconque des revendications précédentes, dans lequel les modules (18, 20) comprennent une liaison mécanique libérable de type à aimantation afin de former une liaison magnétique amovible entre les modules (18, 20).

7. Equipement (10) selon la revendication précédente, dans lequel la liaison magnétique est électriquement conductrice.

8. Equipement (10) selon l'une quelconque des revendications précédentes, dans lequel le module de commande (20) comprend une source (22) d'alimentation électrique reliée à un commutateur (24) marche/arrêt d'activation/désactivation de la source d'alimentation électrique (22), les signaux de commande étant générés par l'activation ou la désactivation de la source d'alimentation électrique (22).

9. Equipement (10) selon la revendication précédente, dans lequel le module de commande (20) comprend des moyens d'ouverture d'un circuit d'alimentation électrique raccordée à la source d'alimentation électrique (22) lorsque les modules lumineux (18) n'émettent pas de lumière.

10. Equipement (10) selon l'une quelconque des revendications précédentes, dans lequel les modules (18, 20) sont aptes à échanger des données par des moyens de communication de données et dans lequel le module de commande (20) comprend des moyens de mémorisation d'un autre programme de traitement prédéfini et des moyens de communication de l'autre programme aux moyens de pilotage (34) des autres modules (18).

11. Equipement (10) selon l'une quelconque des revendications précédentes, dans lequel le module de commande (20) comprend des moyens (102) d'acquisition d'un autre programme de pilotage accessible via un appareil externe, tel qu'un terminal mobile (100).

12. Equipement (10) selon l'une quelconque des revendications précédentes, dans lequel le module de commande (20) comprend une source d'alimentation électrique (22) portative pouvant stocker de l'énergie, en particulier la source est choisie parmi une batterie et une pile à combustible.

13. Equipement (10) selon l'une quelconque des revendications précédentes, dans lequel le module de commande (20) comprend des moyens de communication de données sans fil ou avec fil pour échanger des données avec un appareil externe.

14. Equipement selon l'une quelconque des revendications précédentes, dans lequel les moyens de pilotage sont aptes à piloter la source selon le programme de pilotage comprenant des instructions de code pour la réalisation d'étapes choisies parmi une étape d'allumage de la source de lumière (30), une étape d'extinction de la source de lumière (30), une étape de définition d'un mode d'allumage pulsé ou continu de la source de lumière (30), une étape de sélection d'un spectre d'émission de la source de lumière (30), une étape de définition d'une durée de période d'allumage et une étape de réglage de la puissance d'émission de la source de lumière (30).

15. Equipement (10) selon l'une quelconque des revendications précédentes, dans lequel les moyens de pilotage (34) sont aptes à éteindre la source de lumière (30) en cas d'absence de contact d'une partie du module d'émission de lumière (18) sur un objet externe.
